# EUROPÄISCHE PATENTSCHRIFT

(11) **EP 1 274 673 B1**
(45) Veröffentlichungstag und Bekanntmachung des Hinweises auf die Patenterteilung: **11.10.2006**
(21) Anmeldenummer: 01929466.9
(22) Anmeldetag: 28.03.2001
(51) Int. Cl.: C07C 205/22, C07C 201/08

(54) **VERFAHREN ZUR HERSTELLUNG VON 4-HYDROXY-3-NITROBIPHENYL**
METHOD FOR PRODUCING 4-HYDROXY-3-NITROBIPHENYL
PROCEDE DE PRODUCTION DE 4-HYDROXY-3-NITROBIPHENYLE

(30) Priorität: 10.04.2000 DE 10017818
(43) Veröffentlichungstag der Anmeldung: 15.01.2003
(73) Patentinhaber: Saltigo GmbH, 51369 Leverkusen (DE)
(72) Erfinder: BEHRE, Horst, 51519 Odenthal (DE); DOCKNER, Michael, 50674 Köln (DE); KLAUSENER, Alexander, 50259 Pulheim (DE)
(74) Vertreter: Wichmann, Birgid
(86) Internationale Anmeldenummer: PCT/EP2001/003520
(87) Internationale Veröffentlichungsnummer: WO 2001/077061

(56) Entgegenhaltungen:
- RAIFORD: "3-Nitro-4-Hydroxydiphenyl and some of its derivatives" J. AMER. CHEM. SOC., Bd. 47, 1925, Seiten 1454-1458, XP001013036 in der Anmeldung erwähnt

## Beschreibung

Die vorliegende Erfindung betrifft ein Verfahren zur Herstellung von 4-Hydroxy-3-nitrobiphenyl aus 4-Hydroxybiphenyl durch selektive Nitrierung in Nachbarstellung zur phenolischen Hydroxylgruppe.

4-Hydroxy-3-nitrobiphenyl ist ein wichtiges Vorprodukt für die Herstellung von Pflanzenschutzmitteln. In der Literatur existieren jedoch nur sehr wenige Beispiele für die Herstellung von 4-Hydroxy-3-nitrobiphenyl. Prinzipiell lassen sie sich in zwei Gruppen einteilen.

Zum einen ist es aus Liebigs Ann. Chem. 598, 123 (1955) und DE-A-1 016 716 bekannt, das Biarylsystem photochemisch aus dem 2-Nitro-benzochinon-1,4-diazid-(4) und Benzol aufzubauen. Die Reaktion verläuft jedoch nur mit einer sehr geringen Ausbeute von 25%.

Zum Anderen kann 4-Hydroxybiphenyl durch Nitrierung in 4-Hydroxy-3-nitrobiphenyl überführt werden. Als Nitrierreagentien dienen beispielsweise Silbernitrat, Nitrylchlorid oder Salpetersäure.

Gemäß J. Org. Chem. 32, 300 (1967) wird Silbernitrat mit dem Chloroformiat des 4-Hydroxybiphenyls zu dem entsprechenden Nitrocarbonat umgesetzt. Anschließend erfolgt in einer Umlagerungsreaktion des Intermediats die selektive Einführung der Nitrogruppe in die gewünschte 3-Stellung mit einer Ausbeute von 97 %.

Mit Nitrylchlorid als Nitrierreagenz gelingt die Darstellung des 4-Hydroxy-3-nitrobiphenyls dagegen nur in verunreinigter Form (J. Chem. Soc. Perkin Trans. 2 1985, 2013), wobei die Verunreinigungen nicht spezifiziert werden. Das Vorliegen von Dinitro- und Trinitro-hydroxybiphenyl ist nicht auszuschließen.

Bei Verwendung von Salpetersäure als Nitrierreagenz hängt die Nitrierselektivität sehr stark vom eingesetzten Lösungsmittel ab. Gemäß Chem. Ber. 6, 193 (1873) wird in einem wässrigen System durch Zusammengeben des 4-Hydroxybiphenyls und der Salpetersäure sowie nachfolgendem Erwärmen nicht nur das Mononitrohydroxybiphenyl, sondern ferner das Dinitrohydroxybiphenyl und wohl auch das Trinitrohydroxybiphenyl gebildet. Erst durch Wasserdampfdestillation der Reaktionsmischung wird das 4-Hydroxy-3-nitrobiphenyl in reiner Form erhalten.

Auch FR-A-1.452.911 beschreibt die Umsetzung von 4-Hydroxybiphenyl und verdünnter Salpetersäure in Abwesenheit organischer Lösungsmittel. Bei Zugabe des 4-Hydroxybiphenyls zu einer 50%igen Salpetersäure bei 50°C kommt es vor allem zur Bildung des Dinitrohydroxybiphenyls.

Eine wesentlich höhere Selektivität wird dagegen gemäß J. Am. Chem. Soc. 47, 1454 (1925) durch Nitrieren in Eisessig mit 32 %iger Salpetersäure erzielt. Hierbei wird zunächst das 4-Hydroxybiphenyl in Eisessig gelöst und dann die Salpetersäure zugetropft. Erst danach wird das Reaktionsgemisch erhitzt. Um die gewünschte Selektivität erreichen zu können, ist es jedoch notwendig, mit einer sehr verdünnten Lösung des 4-Hydroxybiphenyls in Eisessig zu arbeiten (1,7 Gew.-% 4-Hydroxybiphenyl). Die Rohausbeute wird mit 80-88 % angegeben, der Zwang zum Arbeiten mit hoher Verdünnung beeinträchtigt die wirtschaftliche Attraktivität des Verfahrens jedoch deutlich.

Alle beschriebenen Methoden zur Herstellung des 4-Hydroxy-3-nitrobiphenyls ausgehend von 4-Hydroxybiphenyl können somit im Hinblick auf eine Durchführung des Verfahrens im technischen Maßstab nicht befriedigen, da eine selektive Einführung der Nitrogruppe in 3-Stellung entweder den Einsatz teurer Nitrierreagenzien voraussetzt oder aber das Arbeiten bei hoher Verdünnung erfordert. Trotz der erzielten, guten bis sehr guten Nitrierselektivität ist zusätzlich eine aufwendige, meist destillative Reinigung erforderlich.

Der Aufbau des Biarylsystems auf photochemischem Weg aus einem nicht käuflichen Edukt gelingt nur mit geringer Ausbeute und erscheint deshalb wenig attraktiv.

Die Aufgabe der vorliegenden Erfindung bestand somit darin, ein Verfahren bereitzustellen, mit dem 4-Hydroxy-3-nitrobiphenyl ausgehend von 4-Hydroxybiphenyl unter Einsatz billiger Reagenzien in einfacher Weise mit hoher Ausbeute und Selektivität hergestellt werden kann.

Gegenstand der Erfindung ist ein Verfahren zur Herstellung von 4-Hydroxy-3-nitrobiphenyl durch Nitrierung von 4-Hydroxybiphenyl mit Salpetersäure, welches dadurch gekennzeichnet ist, dass man eine Mischung aus 4-Hydroxybiphenyl und Eisessig bis zum Sieden des Eisessigs erhitzt und die Salpetersäure oder eine Mischung aus Salpetersäure und Eisessig anschließend in den Rücklauf des siedenden Eisessigs zudosiert.

Mit dem erfindungsgemäßen Verfahren wird das gewünschte 4-Hydroxy-3-nitrobiphenyl mit einer gegenüber dem Stand der Technik verbesserten Ausbeute erhalten. Zudem kann das Arbeiten mit großen Verdünnungen überraschenderweise vermieden werden. Ganz entscheidend für den Erfolg des erfindungsgemäßen Verfahrens ist es, dass die Salpetersäure alleine oder in Mischung mit Eisessig in den Rücklauf des siedenden Eisessigs, insbesondere in den Gasraum (des Eisessigrücklaufs) zugetropft wird. Dies führt zu einer lokalen Verdünnung des Nitrierreagenzes, wodurch ein Arbeiten bei den Konzentrationen des Edukts im Lösungsmittel möglich wird, die deutlich höher liegen als im nächstliegenden Verfahren des Standes der Technik. Ferner kann auf eine Reinigung des Reaktionsproduktes - wie sie nach den Nitrierverfahren des Standes der Technik notwendig ist - verzichtet werden: Das Produkt fällt unmittelbar in einer sehr hohen Reinheit an, die für jede weitere Verwendung geeignet ist.

Im erfindungsgemäßen Verfahren wird zunächst eine Mischung aus 4-Hydroxybiphenyl und Eisessig hergestellt. Der Menge an 4-Hydroxybiphenyl kann dabei 5-50 Gew.-%, bevorzugt 10-30 Gew.-% und besonders bevorzugt 15-20 Gew.-%, bezogen auf die Gesamtmenge aus 4-Hydroxybiphenyl und Eisessig betragen.

Anschließend wird die Mischung aus 4-Hydroxybiphenyl und Eisessig bis zum Sieden des Eisessigs erhitzt. Die Erhitzung der Mischung kann durch Temperaturerhöhung und gegebenenfalls durch gleichzeitige Druckemiedrigung erfolgen. Bevorzugt wird unter Rühren erwärmt, bei gleichzeitiger Verringerung des Drucks. Bevorzugt besitzt die Mischung aus 4-Hydroxybiphenyl und Eisessig einen 4-Hydroxybiphenyl-Gehalt von 15-20 Gew.-% und wird bei einem Druck von 200-300 mbar auf eine Temperatur von 75-80°C erhitzt.

In den Rücklauf des siedenden Eisessigs tropft man Salpetersäure oder eine Mischung aus Eisessig und Salpetersäure. Die Dosiergeschwindigkeit ist abhängig von der Siedetemperatur, der Rückflussgeschwindigkeit des siedenden Eisessigs und der Konzentration des 4-Hydroxybiphenyls im Eisessig und kann in weiten Bereichen variiert werden.

Im Allgemeinen wird mit einer Dosierrate von 0,01 bis 1 g/min, bevorzugt 0,1 bis 0,5 g/min und insbesondere 0,3 bis 0,4 g/min, berechnet für 100 %ige Salpetersäure, Salpetersäure oder eine Mischung aus Eisessig und Salpetersäure in den Rücklauf des siedenden Eisessigs gearbeitet. Die Salpetersäure kann dabei in beliebiger Verdünnung mit Wasser, bevorzugt als 50 bis 70 %ige Salpetersäure und insbesondere als 65 bis 67 %ige Salpetersäure eingesetzt werden. Bewährt hat sich insbesondere der Einsatz von 65 bis 67 %iger Salpetersäure, insbesondere in Mischung mit Eisessig.

Wird eine Mischung aus Eisessig und Salpetersäure zugetropft, so haben sich 1:10 bis 10:1-Mischungen bewährt. Bevorzugt wird eine 7:3 bis 3:7 und insbesondere eine 1:1 Mischung aus Eisessig und 65 bis 67 %iger Salpetersäure eingesetzt.

Die Menge der alleine oder in Mischung mit dem Eisessig zugetropften Salpetersäure wird so bemessen, dass das molare Verhältnis von Salpetersäure zu eingesetztem 4-Hydroxybiphenyl 1,2:1 bis 0,8:1, bevorzugt 1,05:1 bis 1:1 beträgt.

Nach Beendigung des Zutropfens wird die Reaktionsmischung noch eine gewisse Zeit nachgerührt. Bewährt hat sich eine Nachrührzeit von 10 Minuten bis zu einer Stunde, bevorzugt 10 Minuten bis zu 30 Minuten, insbesondere ca. 15 Minuten bei einer Temperatur, bei der der Eisessig unter den gegebenen Druckbedingungen weiterhin siedet. Anschließend wird das Reaktionsgemisch zu Wasser hinzugegeben.

Sowohl die Nachrührzeit, als auch die Temperatur der Reaktionsmischung zum Zeitpunkt der Zugabe zum Wasser, als auch die Temperatur des Wassers sind nicht essentiell für die im erfindungsgemäßen Verfahren erzielte Selektivität.

In einer Variante des erfindungsgemäßen Verfahrens ist es auch möglich, die Reaktionsmischung zunächst aufzukonzentrieren und erst danach auf Wasser zu geben.

Wenn man das Reaktionsgemisch zu dem Wasser gibt, fällt das gewünschte 4-Hydroxy-3-nitrobiphenyl als Niederschlag aus und kann in einfacher Weise durch Filtration abgetrennt werden, mit Wasser ein- oder mehrmals gewaschen und an der Luft getrocknet werden.

### Beispiele:

### Beispiel 1:

In einem 2 l Doppelmantelplanschliffbecher mit Glasimpellerrührer, Thermometer und Rückflusskühler mit Einleitrohr werden 200 g (1,18 mol) 4-Hydroxybiphenyl und 1000 g Eisessig vorgelegt. In der Apparatur wird ein Vakuum von 240 mbar erzeugt und die Mischung auf 80°C zum kräftigen Sieden des Eisessigs erwärmt. Unter Rühren wird innerhalb von vier Stunden eine Mischung aus 116,8 g Eisessig und 116,8 g Salpetersäure (65 Gew.-%, 1,21 mol) über ein Einleitrohr aus Teflon, das von oben in den Rückflusskühler eingeführt wird und dessen Öffnung sich auf der Höhe des letzten Drittels der Gesamtrückflusskühlerlänge befindet, bei 75-80°C zugegeben. Nach beendeter Zugabe wird noch 15 Minuten gerührt. Man kühlt die Reaktionsmischung auf 40°C ab und belüftet die Apparatur mit Stickstoff.

Anschließend wird der Reaktionsansatz innerhalb von 20 Minuten auf 3350 g Wasser bei 40°C gegeben. Die Reaktionsmischung wird in eineinhalb Stunden auf 20°C abgekühlt, der ausgefallene Feststoff abfiltriert und zweimal mit jeweils 500 g Wasser gewaschen. Danach wird das Produkt über Nacht bei 20°C im Luftstrom getrocknet. Es werden 249,6 g 4-Hydroxy-3-nitrobiphenyl erhalten.

Mittels HPLC werden die folgenden Flächen-% erhalten, aus denen unter Verwendung eines Standards die zugehörigen Gew.%-Angaben ermittelt werden:

| | **Flächen-%** | **Gew.-%** |
|---|---|---|
| 4-Hydroxy-3-nitrobiphenyl | 99,3 | 94,2 |
| 4-Hydroxbiphenyl | 0,1 | 0,1 |
| 3,5-Dinitro-4-hydroxybiphenyl | 0,3 | 0,3 |
| unbekannte Verunreinigung bei einer Retentionszeit von 16,9 min | 0,1 | -- |
| unbekannte Verunreinigung bei einer Retentionszeit von 17,8 min | 0,1 | -- |

Bei den zu einem Wert von 100 fehlenden Flächen-% handelt es sich um Eisessig.

Die Ausbeute an 4-Hydroxy-3-nitrobiphenyl beträgt damit 93 % der Theorie, bezogen auf eingesetztes 4-Hydroxybiphenyl.

### Beispiel 2

### (Vergleichsversuch gemäß Beispiel in J. Am. Chem. Soc. 47, 1454 (1925) mit einer Eduktkonzentration von 1,7 Gew.-%)

In einem Vierhalskolben mit Rückflusskühler, Thermometer, Rührer und Tropftrichter werden 4,5 g (0,026 mol) 4-Hydroxybiphenyl in 250 ml Eisessig vorgelegt. Unter Rühren tropft man innerhalb von 90 Minuten 4,96 g (0,026 mol) einer 33 gew.%igen Salpetersäure zu und erwärmt die Mischung anschließend auf 80°C. Nach 2,5 Stunden bei 80 °C wird die Reaktionsmischung auf 750 g einer Eis/Wasser-Mischung gegeben und kurz verrührt. Der gebildete Feststoff wird abfiltriert, mit kaltem Wasser gewaschen und über Phosphorpentoxid im Vakuum getrocknet.

Es werden 5,1 g 4-Hydroxy-3-nitrobiphenyl erhalten. Dies entspricht einer Rohausbeute von 91 % der Theorie.

Mittels HPLC werden die folgenden Flächen-% erhalten, aus denen unter Verwendung eines Standards die zugehörigen Gew.%-Angaben ermittelt werden:

| | **Flächen-%** | **Gew.-%** |
|---|---|---|
| 4-Hydroxy-3-nitrobiphenyl | 97,2 | 91,1 |
| 4-Hydroxbiphenyl | 0,5 | 0,6 |
| 3,5-Dinitro-4-hydroxybiphenyl | nicht vorhanden | -- |
| unbekannte Verunreinigung bei einer Retentionszeit von 16,9 min | 0,4 | -- |
| unbekannte Verunreinigung bei einer Retentionszeit von 17,8 min | 1,6 | -- |

Bei den zu einem Wert von 100 fehlenden Flächen-% handelt es sich um Eisessig.

Die Ausbeute an 4-Hydroxy-3-nitrobiphenyl beträgt damit 100 % der Theorie, bezogen auf eingesetztes 4-Hydroxybiphenyl.
Es wird ein Schmelzpunkt von 67,5 °C gemessen.

### Beispiel 3

### (Vergleichsversuch gemäß Beispiel in J. Am. Chem. Soc. 47, 1454 (1925), aber mit einer Eduktkonzentration von 17 Gew.% statt 1,7 Gew.%)

In einem Vierhalskolben mit Rückflusskühler, Thermometer, Rührer und Tropftrichter werden 53,8 g (0,316 mol) 4-Hydroxybiphenyl in 250 ml Eisessig vorgelegt. Unter Rühren tropft man innerhalb von 90 Minuten 60,3 g (0,316 mol) einer 33 gew.%igen Salpetersäure zu und erwärmt die Mischung anschließend auf 80°C. Nach 5 Stunden bei 80°C wird die Reaktionsmischung auf 750 g einer Eis/Wasser-Mischung gegeben und kurz verrührt. Der gebildete Feststoff wird abfiltriert, mit kaltem Wasser gewaschen und über Nacht bei 20°C im Luftstrom getrocknet.

Es werden 67,8 g 4-Hydroxy-3-nitrobiphenyl erhalten. Dies entspricht einer Rohausbeute von 99 %.

Mittels HPLC werden die folgenden Flächen-% erhalten, aus denen unter Verwendung eines Standards die zugehörigen Gew.%-Angaben ermittelt werden:

| | **Flächen-%** | **Gew.-%** |
|---|---|---|
| 4-Hydroxy-3-nitrobiphenyl | 95,6 | 79,0 |
| 4-Hydroxbiphenyl | 2,3 | 2,2 |
| 3,5-Dinitro-4-hydroxybiphenyl | nicht vorhanden | -- |
| unbekannte Verunreinigung bei einer Retentionszeit von 16,9 min | 1,1 | -- |
| unbekannte Verunreinigung bei einer Retentionszeit von 17,8 min | 0,3 | -- |

Bei den zu einem Wert von 100 fehlenden Flächen-% handelt es sich um Eisessig.

Die Ausbeute an 4-Hydroxy-3-nitrobiphenyl beträgt damit 79 % der Theorie, bezogen auf eingesetztes 4-Hydroxybiphenyl.

## Patentansprüche

1. Verfahren zur Herstellung von 4-Hydroxy-3-nitrobiphenyl durch Nitrierung von 4-Hydroxybiphenyl mit Salpetersäure, **dadurch gekennzeichnet, dass** man eine Mischung aus 4-Hydroxybiphenyl und Eisessig bis zum Sieden des Eisessigs erhitzt und die Salpetersäure oder eine Mischung aus Salpetersäure und Eisessig anschließend in den Rücklauf des siedenden Eisessigs zudosiert.

2. Verfahren nach Anspruch 1, **dadurch gekennzeichnet, dass** die eingesetzte Mischung aus 4-Hydroxybiphenyl und Eisessig einen 4-Hydroxybiphenyl-Gehalt von 5 bis 50 Gew.-%, bevorzugt 10 bis 30 Gew.-% und besonders bevorzugt 15 bis 20 Gew.-% besitzt.

3. Verfahren nach Anspruch 1 oder 2, **dadurch gekennzeichnet, dass** die Erhitzung der Mischung aus 4-Hydroxybiphenyl und Eisessig durch Temperaturerhöhung und gegebenenfalls gleichzeitige Druckerniedrigung erfolgt.

4. Verfahren nach einem oder mehreren der Ansprüche 1 bis 3, **dadurch gekennzeichnet, dass** die Mischung aus 4-Hydroxybiphenyl und Eisessig einen 4-Hydroxybiphenyl-Gehalt von 15 bis 20 Gew.% besitzt und die Mischung bei einem Druck von 200 bis 300 mbar auf eine Temperatur von 75 bis 80°C erhitzt wird.

5. Verfahren nach einem oder mehreren der Ansprüche 1 bis 4, **dadurch gekennzeichnet, dass** eine mit Wasser verdünnte Salpetersäure, bevorzugt eine 50 bis 70 %ige Salpetersäure und insbesondere eine 65 bis 67 %ige Salpetersäure verwendet wird.

6. Verfahren nach einem oder mehreren der Ansprüche 1 bis 5, **dadurch gekennzeichnet, dass** mit einer Dosierrate von 0,01 bis 1 g/min, bevorzugt 0,1 bis 0,5 g/min und insbesondere 0,3 bis 0,4 g/min, berechnet für 100 %ige Salpetersäure, Salpetersäure oder eine Mischung aus Salpetersäure und Eisessig in den Rücklauf des siedenden Eisessigs gearbeitet wird.

7. Verfahren nach einem oder mehreren der Ansprüche 1 bis 6, **dadurch gekennzeichnet, dass** 1:10 bis 10:1-Mischungen, bevorzugt eine 7:3 bis 3:7 und insbesondere eine 1:1 Mischung aus Eisessig und 65 bis 67 %iger Salpetersäure eingesetzt werden.

8. Verfahren nach einem oder mehreren der Ansprüche 1 bis 7, **dadurch gekennzeichnet, dass** die Menge der alleine oder in Mischung mit dem Eisessig zugetropften Salpetersäure so bemessen wird, dass das molare Verhältnis von Salpetersäure zu 4-Hydroxybiphenyl 1,2:1 bis 0,8:1, bevorzugt 1,05:1 bis 1:1 beträgt.

9. Verfahren nach einem oder mehreren der Ansprüche 1 bis 8, **dadurch gekennzeichnet, dass** das Reaktionsgemisch nach der Umsetzung auf Wasser gegeben wird, das anfallende 4-Hydroxy-3-nitrobiphenyl abfiltriert, gegebenenfalls mit Wasser gewaschen und getrocknet wird.

## Claims

1. Process for preparing 4-hydroxy-3-nitrobiphenyl by nitrating 4-hydroxybiphenyl using nitric acid, **characterized in that** a mixture of 4-hydroxybiphenyl and glacial acetic acid is heated until the glacial acetic acid boils and the nitric acid or a mixture of nitric acid and glacial acetic acid is then metered into the reflux of the boiling glacial acetic acid.

2. Process according to Claim 1, **characterized in that** the mixture of 4-hydroxybiphenyl and glacial acetic acid used has a 4-hydroxybiphenyl content of from 5 to 50% by weight, preferably from 10 to 30% by weight and more preferably from 15 to 20% by weight.

3. Process according to Claim 1 or 2, **characterized in that** the mixture of 4-hydroxybiphenyl and glacial acetic acid is heated by increasing the temperature and optionally by reducing the pressure at the same time.

4. Process according to one or more of Claims 1 to 3, **characterized in that** the mixture of 4-hydroxybiphenyl and glacial acetic acid has a 4-hydroxybiphenyl content of from 15 to 20% by weight and the mixture is heated at a pressure of from 200 to 300 mbar to a temperature of from 75 to 80°C.

5. Process according to one or more of Claims 1 to 4, **characterized in that** the nitric acid used is diluted with water, and is preferably from 50 to 70% nitric acid and in particular from 65 to 67% nitric acid.

6. Process according to one or more of Claims 1 to 5, **characterized in that** nitric acid or a mixture of nitric acid and glacial acetic acid is metered into the reflux of the boiling glacial acetic acid at a rate of from 0.01 to 1 g/min, preferably from 0.1 to 0.5 g/min and in particular from 0.3 to 0.4 g/min, calculated for 100% nitric acid.

7. Process according to one or more of Claims 1 to 6, **characterized in that** from 1:10 to 10:1 mixtures, preferably 7:3 to 3:7 mixtures and in particular a 1:1 mixture of glacial acetic acid and from 65 to 67% nitric acid are used.

8. Process according to one or more of Claims 1 to 7, **characterized in that** nitric acid is added dropwise alone or in a mixture with the glacial acetic acid in such an amount that the molar ratio of nitric acid to 4-hydroxybiphenyl is from 1.2:1 to 0.8:1, preferably from 1.05:1 to 1:1.

9. Process according to one or more of Claims 1 to 8, **characterized in that** the reaction mixture after the reaction is added to water, the resulting 4-hydroxy-3-nitrobiphenyl is filtered off, optionally washed with water and dried.

## Revendications

1. Procédé de préparation de 4-hydroxy-3-nitrobiphényle par nitration de 4-hydroxybiphényle avec de l'acide nitrique, **caractérisé en ce qu'**un mélange de 4-hydroxybiphényle et d'acide acétique glacial est chauffé jusqu'à l'ébullition de l'acide acétique glacial et l'acide nitrique ou un mélange d'acide nitrique et d'acide acétique glacial est ensuite ajouté en quantité mesurée au reflux de l'acide acétique glacial à ébullition.

2. Procédé selon la revendication 1, **caractérisé en ce que** le mélange utilisé de 4-hydroxybiphényle et d'acide acétique glacial présente une teneur en 4-hydroxybiphényle de 5 à 50 % en poids, de préférence de 10 à 30 % en poids, et particulièrement préférablement de 15 à 20 % en poids.

3. Procédé selon la revendication 1 ou 2, **caractérisé en ce que** le chauffage du mélange de 4-hydroxybiphényle et d'acide acétique glacial est réalisé par augmentation de la température et éventuellement par réduction simultanée de la pression.

4. Procédé selon l'une ou plusieurs des revendications 1 à 3, **caractérisé en ce que** le mélange de 4-hydroxybiphényle et d'acide acétique glacial présente une teneur en 4-hydroxybiphényle de 15 à 20 % en poids, et le mélange est chauffé à une température de 75 à 80°C à une pression de 200 à 300 mbars.

5. Procédé selon l'une ou plusieurs des revendications 1 à 4, **caractérisé en ce que** l'on utilise un acide nitrique dilué à l'eau, de préférence un acide nitrique à 50 à 70 %, et en particulier un acide nitrique à 65 à 67 %.

6. Procédé selon l'une ou plusieurs des revendications 1 à 5, **caractérisé en ce que** l'on travaille avec un taux d'addition de 0,01 à 1 g/min, de préférence de 0,1 à 0,5 g/min, et en particulier de 0,3 à 0,4 g/min, calculé pour de l'acide nitrique à 100 %, de l'acide nitrique ou un mélange d'acide nitrique et d'acide acétique glacial, au reflux de l'acide acétique glacial.

7. Procédé selon l'une ou plusieurs des revendications 1 à 6, **caractérisé en ce que** l'on utilise des mélanges à 1:10 à 10:1, de préférence un mélange à 7:3 à 3:7, et en particulier un mélange à 1:1 d'acide acétique glacial et d'acide nitrique à 65 à 67 %.

8. Procédé selon l'une ou plusieurs des revendications 1 à 7, **caractérisé en ce que** la quantité de l'acide nitrique seul ou ajouté goutte à goutte en mélange avec l'acide acétique glacial est mesurée de telle sorte que le rapport molaire entre l'acide nitrique et le 4-hydroxybiphényle soit de 1,2:1 à 0,8:1, de préférence de 1,05:1 à 1:1.

9. Procédé selon l'une ou plusieurs des revendications 1 à 8, **caractérisé en ce que** le mélange réactionnel est ajouté dans de l'eau après la réaction, le 4-hydroxy-3-nitrobiphényle formé est filtré, éventuellement lavé avec de l'eau et séché.
